# EUROPEAN PATENT APPLICATION

(11) **EP 4 029 555 A1**
(43) Date of publication of application: **20.07.2022**
(21) Application number: 20864160.5
(22) Date of filing: 04.09.2020
(51) Int. Cl.: A61M 25/06, A61M 25/00, A61M 25/088

(54) **SUPPORT CATHETER AND TUBE**

(30) Priority: 11.09.2019 JP 2019165242; 11.09.2019 JP 2019165243
(71) Applicant: Nipro Corporation, Osaka-shi, Osaka 531-8510 (JP)
(72) Inventor: KAMEOKA, Koji, Osaka-shi Osaka 531-8510 (JP); MORI, Yoshihiro, Osaka-shi Osaka 531-8510 (JP)
(74) Representative: Dantz, Jan Henning
(86) International application number: PCT/JP2020/033567
(87) International publication number: WO 2021/049431

(57) **Abstract**

A support catheter includes: a distal shaft shaped as a tube into which a therapeutic catheter is insertable, the distal shaft including an inner layer and a reinforcing layer, the reinforcing layer being shaped as a tubular mesh including metal wires wound in first and second opposite directions; and a proximal shaft connected to the distal shaft. The reinforcing layer includes welds at intersections of the metal wires wound in the first and second directions. The welds are located in a limited region in a circumferential direction of the distal shaft. A covering member is located outside the welds.

## Description

### Technical Field

The present invention relates to a support catheter used with a therapeutic catheter and a guiding catheter to guide the therapeutic catheter to a treatment site, and further relates to a tube.

### Background Art

A description concerning the first disclosure of the invention will be given first. In percutaneous coronary intervention (PCI), a support catheter may be used with a therapeutic catheter and a guiding catheter.

The support catheter includes a distal shaft shaped as a tube into which the therapeutic catheter is insertable and a proximal shaft connected to the distal shaft. In some cases, the distal shaft includes a tube including a metal braid serving as a reinforcing layer to ensure pushability and kink resistance.

The wires forming the metal braid are likely to expand radially outward at their cut ends. Thus, a structure is conventionally used in which the ends of the wires are welded together and in which the welds are covered by an outer layer.

With the aim of preventing the outer layer from bulging at the locations of the covered welds or preventing the welds from sticking out from the outer layer, a catheter has been proposed which includes a thin intermediate layer made of a resin and located between the outer circumference of a metal braid and the inner circumferential surface of an outer tube. Such a catheter is disclosed, for example, in Patent Literature 1.

A description is given below of the second disclosure of the invention. A support catheter includes: a distal shaft shaped as a tube into which a therapeutic catheter is insertable, the distal shaft including an inner layer, a reinforcing layer, and an outer layer; and a proximal shaft connected to the distal shaft. The reinforcing layer may include a tubular braid formed of metal wires or resin wires wound with a constant pitch (see Patent Literature 1, for example).

### Citation List

### Patent Literature

PTL 1: Japanese Laid-Open Patent Application Publication No. H9-294810
PTL 2: Japanese Laid-Open Patent Application Publication No. 2007-82802

### Summary of Invention

### Technical Problem

Regarding the first disclosure, the intermediate layer of Patent Literature 1 extends over the entire circumference of the metal braid to cover the welds, and thus could cause an increase in the outer diameter of the catheter.

It is therefore an object of the present invention to provide a support catheter and a tube in which the increase in outer diameter can be reduced or avoided and in which radial sticking out of welds of metal wires (wires) of a reinforcing layer can be reduced or prevented.

Regarding the second disclosure, one possible way of connecting the proximal and distal shafts is to fix one end portion of the proximal shaft onto the reinforcing layer (braid) of the distal shaft and thereby connect the shafts together.

However, in the case where one end portion of a proximal shaft is fixed onto a braid which is formed of wires wound with a constant pitch as in Patent Literature 1, a longitudinal tensile force acting on a distal shaft imposes a load on the point at which the one end portion of the proximal shaft and the braid (wires) are fixed together. This load could cause the one end portion of the proximal shaft to be detached from the wires and the distal end of the proximal shaft to stick out from the outer layer.

Therefore, a problem to be solved by the present invention is how to provide a support catheter having a novel structure in which one end portion of a proximal shaft is resistant to detachment from a braid of a distal shaft.

Another problem to be solved by the present invention is how to provide a tube in which a member fixed to a braid is resistant to detachment from the braid.

### Solution to Problem

The following describes the first disclosure. A support catheter according to a first aspect of the first disclosure is for use with a therapeutic catheter for treating a treatment site and a guiding catheter for receiving insertion of the therapeutic catheter and guiding the therapeutic catheter in a blood vessel, the support catheter being long enough to project out of a distal end opening of the guiding catheter when inserted into the guiding catheter through a proximal end opening of the guiding catheter, the support catheter being adapted to guide a distal portion of the therapeutic catheter to the treatment site, the support catheter including: a distal shaft shaped as a tube into which the therapeutic catheter is insertable, the distal shaft including an inner layer and a reinforcing layer, the reinforcing layer being shaped as a tubular mesh including metal wires wound in first and second opposite directions; and a proximal shaft connected to the distal shaft, wherein the reinforcing layer includes welds at intersections of the metal wires wound in the first and second directions, the welds are located in a limited region in a circumferential direction of the distal shaft, and a covering member is located outside the welds.

According to the first aspect, since the covering member is located outside the welds, the welds are covered by the covering member directly or indirectly and less likely to stick out radially from the covering member. Additionally, the welds are located in a limited region in the circumferential direction of the distal shaft. Thus, unlike in conventional structures, there is no need for any intermediate layer located between the inner and outer layers and extending over the entire circumference of the distal shaft, and the covering member is placed only over the limited region where the welds are located. As such, the increase in diameter of the distal shaft can be reduced or avoided. This allows for reducing or avoiding the increase in outer diameter of the support catheter.

The second aspect of the first disclosure is directed to the support catheter according to the first aspect of the first disclosure, wherein the covering member includes at least one of a cover piece and a distal portion of the proximal shaft made of a metal.

According to the second aspect, it is possible to increase the strength of the distal shaft as well as to reduce or avoid sticking out of the welds in the case where the distal portion of the proximal shaft is used as the covering member, while in the case where the cover piece is used as the covering member, it is possible to ensure the flexibility of the distal shaft as well as to reduce or avoid sticking out of the welds. Additionally, the hardness of the distal shaft can be adjusted by adjusting the areas over which the distal portion of the proximal shaft and the cover piece are located, such as by placing the distal portion of the proximal shaft outside some of the welds and placing the cover piece outside the other welds.

A third aspect of the first disclosure is directed to the support catheter according to the first aspect of the first disclosure, wherein the welds are arranged in a single row extending in an axial direction of the distal shaft.

According to the third aspect, since the welds are arranged in a single row extending in the axial direction of the distal shaft, all of the welds can easily be covered by a substantially strip-shaped distal portion of the proximal shaft or a substantially strip-shaped cover piece. Additionally, since the covering member is placed only over a limited region, the increase in outer diameter of the distal shaft and therefore the increase in outer diameter of the support catheter can be further reduced.

A fourth aspect of the first disclosure is directed to the support catheter according to the first aspect of the first disclosure, wherein the welds are arranged in two rows extending in an axial direction of the distal shaft.

According to the fourth aspect, since the welds are arranged in two separate rows, the length of the covering member in the axial direction of the distal shaft can be reduced. This can ensure the flexibility of the distal shaft.

A fifth aspect of the first disclosure is directed to the support catheter according to the first aspect of the first disclosure, wherein one of the two rows is 180° away from the other row in a circumferential direction of the inner layer.

According to the fifth aspect, the distal shaft can be shaped to have a slim, elliptical outline, and the distal shaft thus shaped allows for easy insertion of a medical instrument into the guiding catheter. Additionally, the welding operation is easy to perform since the welds of the metal wires are arranged in discrete rows and prevented from being too close to each other in the circumferential direction of the covering member.

A sixth aspect of the first disclosure is directed to the support catheter according to the first aspect of the first disclosure, wherein one of the two rows is 90° away from the other row in a circumferential direction of the inner layer.

According to the sixth aspect, both the length of the covering member in the axial direction of the distal shaft and the length of the covering member in the circumferential direction of the distal shaft can be reduced, and thus the flexibility of the distal shaft can be ensured. Additionally, the welding operation is easy to perform since the welds of the metal wires are arranged in discrete rows and prevented from being too close to each other in the circumferential direction of the covering member.

A tube according to a seventh aspect of the first disclosure includes: a tubular inner layer; and a reinforcing layer located on an outer surface of the inner layer and shaped as a tubular mesh including metal wires wound in first and second opposite directions, wherein the reinforcing layer includes welds at intersections of the metal wires wound in the first and second directions, the welds are located in a limited region in a circumferential direction of the reinforcing layer, and a covering member is located outside the welds.

According to the seventh aspect, since the covering member is located outside the welds, the welds are covered by the covering member directly or indirectly and less likely to stick out radially from the covering member. Additionally, the welds are located in a limited region in the circumferential direction of the reinforcing layer. Thus, unlike in conventional structures, there is no need for any intermediate layer located between the inner and outer layers and extending over the entire circumference of the tube, and the covering member is placed only over the limited region where the welds are located. As such, the increase in diameter of the tube can be reduced or avoided.

An eighth aspect of the first disclosure is directed to the tube according to the seventh aspect of the first disclosure, wherein the welds are arranged in a single row extending in an axial direction of the inner layer.

According to the eighth aspect, since the welds are arranged in a single row extending in the axial direction of the distal shaft, all of the welds can easily be covered by a substantially strip-shaped covering member. Additionally, since the covering member is placed only over a limited region, the increase in outer diameter of the tube can be further reduced.

The following describes the second disclosure. A support catheter according to a first aspect of the second disclosure is a support catheter for use with a therapeutic catheter for treating a treatment site and a guiding catheter for receiving insertion of the therapeutic catheter and guiding the therapeutic catheter in a blood vessel, the support catheter being long enough to project out of a distal end opening of the guiding catheter when inserted into the guiding catheter through a proximal end opening of the guiding catheter, the support catheter being adapted to guide a distal portion of the therapeutic catheter to the treatment site, the support catheter including: a distal shaft shaped as a tube into which the therapeutic catheter is insertable, the distal shaft including an inner layer and a reinforcing layer, the reinforcing layer being shaped as a tubular mesh including metal wires wound in first and second opposite directions; and a proximal shaft connected to the distal shaft, wherein the reinforcing layer includes a short pitch portion in which a pitch of the wound metal wires is a first value and a long pitch portion in which the pitch of the wound metal wires is a second value greater than the first value, and one end portion of the proximal shaft is fixed to the long pitch portion.

According to the first aspect, the reinforcing layer includes the long pitch portion, and one end portion of the proximal shaft is fixed to the long pitch portion. Thus, in the event that a longitudinal tensile force acts on the distal shaft, the load imposed on the fixing points can be lower than in the case where the one end portion of the proximal shaft is fixed to the short pitch portion.

In the case where one end portion of a proximal shaft is fixed to a braid of a distal shaft, the angle of metal wires of the braid is fixed at a given value in that portion of the braid to which the one end portion of the proximal shaft is fixed. In the event that a conventional distal shaft including a reinforcing layer in the form of a braid made of wires wound with a constant pitch is pulled in the longitudinal direction of the distal shaft, the portion of the braid that is not fixed by the proximal shaft is gradually deformed in such a direction that the pitch increases (the angle of the metal wires increases), but the portion of the braid that maintains a fixed angle of the metal wires cannot conform to the above deformation. Thus, a load is imposed on a fixing point which is at the boundary between the pitch variable portion where the braid pitch changes and the pitch invariable portion where the pitch remains unchanged. In the support catheter according to the first aspect, the portion of the distal shaft that is fixed by the proximal shaft has a longer braid pitch (greater angle of the metal wires) than the rest of the distal shaft. Thus, even when the distal shaft is pulled in the longitudinal direction, the load imposed on fixing points due to changes occurring in the portion of the braid that is not fixed by the proximal shaft (changes in the braid pitch and the angle of the metal wires) can be reduced. This makes the one end portion of the proximal shaft resistant to detachment of from the metal wires.

A second aspect of the second disclosure is directed to the support catheter according to the first aspect of the second disclosure, wherein the reinforcing layer includes two short pitch portions in which the pitch of the wound metal wires is the first value, and the long pitch portion is located between the two short pitch portions.

According to the second aspect, the long pitch portion harder than the short pitch portions can be located in a limited region to which the one end portion of the proximal shaft is fixed, and one of the short pitch portions can be proximal to the limited region. This ensures the flexibility of the distal shaft, thus preventing breakage of the distal shaft.

A third aspect of the second disclosure is directed to the support catheter according to the first aspect of the second disclosure, wherein the reinforcing layer includes a pitch-changing portion between the short pitch portion and the long pitch portion, and in the pitch-changing portion, the pitch of the wound metal wires decreases in a direction from the long pitch portion to the short pitch portion.

According to the third aspect, since the braid pitch changes gradually between the long pitch portion and the short pitch portion, an abrupt hardness change of the distal shaft can be avoided to prevent breakage of the distal shaft. The pitch-changing portion is preferably short in order to ensure the flexibility of the distal shaft and preferably long in order to moderate the change in hardness of the distal shaft.

A fourth aspect of the second disclosure is directed to the support catheter according to the first aspect of the second disclosure, wherein the one end portion of the proximal shaft is welded to the long pitch portion at two or more of axially aligned intersections of the metal wires wound in the first and second directions.

According to the fourth aspect, the one end portion of the proximal shaft is fixed to the long pitch portion at two or more points. Thus, even in the event that the one end portion of the proximal shaft is detached from the distal fixing point subjected to the highest load, the proximal shaft and the distal shaft are not readily separated from each other.

A fifth aspect of the second disclosure is directed to the support catheter according to the first aspect of the second disclosure, wherein when the support catheter is in a normal state, an acute angle between the wound metal wire of the long pitch portion and a straight line perpendicular to a longitudinal direction of the inner layer is from 25° to 70°.

According to the fifth aspect, the closer the acute angle is to 90°, the higher the longitudinal tensile strength of the distal shaft is. The closer the acute angle is to 0°, the higher the flexibility of the distal shaft is, and the easier it is to move the distal shaft radially.

A tube according to the sixth aspect of the second disclosure includes: a tubular inner layer; and a reinforcing layer located on an outer surface of the inner layer and shaped as a tubular mesh including metal wires wound in first and second opposite directions, wherein the reinforcing layer includes a short pitch portion in which a pitch of the wound metal wires is a first value and a long pitch portion in which the pitch of the wound metal wires is a second value greater than the first value, and the tube further includes a fixed member fixed to the long pitch portion.

According to the sixth aspect, the reinforcing layer includes the long pitch portion, and the fixed member is fixed to the long pitch portion. Thus, in the event that a longitudinal tensile force acts on the tube, the load imposed on the fixing points can be lower than in the case where one end portion of the fixed member is fixed to the short pitch portion. As such, the load imposed on the fixing points is reduced as described above for the first aspect, and this makes the fixed member resistant to detachment from the metal wires (braid).

### Advantageous Effects of Invention

Regarding the first disclosure, the present invention can provide a support catheter and a tube in which the increase in outer diameter can be reduced or avoided and in which the radial sticking out of welds of metal wires of a reinforcing layer can be reduced or prevented. Regarding the second disclosure, the present invention can provide: a support catheter having a novel structure in which one end portion of a proximal shaft is resistant to detachment from a braid of a distal shaft; and a tube in which a member fixed to a braid is resistant to detachment from the braid.

### Brief Description of Drawings

FIG. 1 relates to the first and second disclosures and shows how a support catheter according to an embodiment of the present invention is used with a therapeutic catheter and a guiding catheter.
FIG. 2 relates to the first disclosure and is a side view showing the support catheter of FIG. 1.
FIG. 3A relates to the first disclosure and shows the arrangement of welds of metal wires in a first embodiment, and FIG. 3B is a plan view showing the proximal shaft of FIG. 3A.
FIG. 4 relates to the first disclosure and shows locations on the inner layer at which the welds are located.
FIG. 5A relates to the first disclosure and is a perspective view showing a cover piece, and FIG. 5B is a perspective view showing a variant of the cover piece.
FIG. 6A relates to the first disclosure and shows the arrangement of welds of metal wires in a second embodiment, and FIG. 6B shows the arrangement of the welds when the FIG.6A is rotated by 90° around the axis.
FIG. 7A relates to the first disclosure and shows the arrangement of welds of metal wires in a third embodiment, and FIG. 7B shows the arrangement of the welds when the FIG.7A is rotated by 90° around the axis.
FIG. 8A relates to the first disclosure and shows the arrangement of welds of metal wires in a fourth embodiment, and FIG. 8B shows the arrangement of the welds when the FIG.8A is rotated by 45° around the axis.
FIG. 9A relates to the first disclosure and shows the arrangement of welds of metal wires in a fifth embodiment, and FIG. 9B shows the arrangement of the welds when the FIG.9A is rotated by 180° around the axis.
FIG. 10 relates to the first disclosure and shows the arrangement of welds of metal wires in a sixth embodiment.
FIG. 11 relates to the first disclosure and is a side view showing a tube according to an embodiment of the present invention.
FIG. 12 relates to the second disclosure and is a side view showing the support catheter of FIG. 1.
FIG. 13 relates to the second disclosure and is a photograph of the distal shaft having no outer layer.
FIG. 14 relates to the second disclosure and shows the distal shaft having no outer layer.
FIG. 15 relates to the second disclosure and is a graph showing the relationship between the braid pitch and metal wire angle in a long pitch portion.
FIG. 16 relates to the second disclosure and shows fixing points at which one end portion of the proximal shaft is fixed to the reinforcing layer.
FIG. 17A relates to the second disclosure and shows a reinforcing layer of a conventional support catheter, and FIG. 17B shows the reinforcing layer as viewed when the proximal shaft of FIG. 17A is being pulled.
FIG. 18A relates to the second disclosure and shows a reinforcing layer of a support catheter according to an embodiment of the present invention, and FIG. 18B shows the reinforcing layer as viewed when the proximal shaft of FIG. 18A is being pulled.
FIG. 19 relates to the second disclosure and is a side view showing a tube according to an embodiment of the present invention.

### Description of Embodiments

Hereinafter, support catheters according to embodiments of the present invention will be described with reference to the drawings. The support catheters described below are merely exemplary embodiments of the present invention. The present invention is not limited to the embodiments described below, and additions, deletions, and changes can be made without departing from the gist of the present invention. The directions mentioned in the following description are used merely for convenience of illustration and are not intended to limit the positions or orientations of the elements of the invention.

### (First embodiment according to first disclosure)

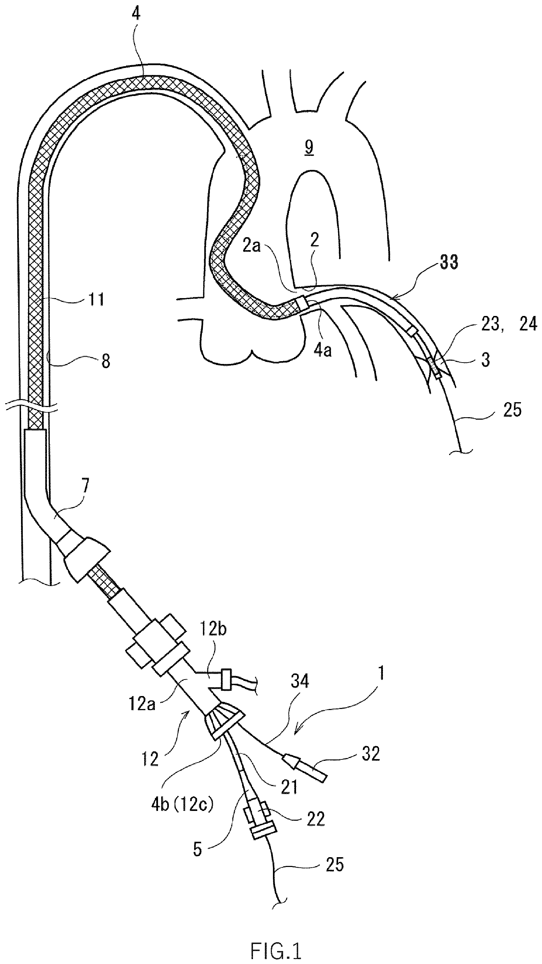

A known example of techniques for dilating a stenosed region 3 of a coronary artery 2 shown in FIG. 1 is percutaneous coronary intervention (PCI). The PCI is performed typically using a guiding catheter 4, a balloon catheter 5, a support catheter 1 of the present embodiment, and a guide wire 25. The following describes the instruments used in the PCI.

### <Guiding catheter>

The guiding catheter 4 is a catheter for guiding the balloon catheter 5 and the support catheter 1 in a blood vessel. The guiding catheter 4 is inserted, for example, into a radial artery 8 or a non-illustrated femoral artery through a sheath 7. The guiding catheter 4 includes a guiding catheter main body 11 and a Y-shaped connector 12. The guiding catheter main body 11 is shaped as an elongated tube, and the balloon catheter 5 and support catheter 1 are insertable into the guiding catheter main body 11. The guiding catheter main body 11 is a bendable, cylindrical flexible tube and can be advanced inside winding blood vessels.

The Y-shaped connector 12 is located at the proximal end of the guiding catheter main body 11. The Y-shaped connector 12 includes a main body 12a and a side arm 12b, and a drug solution or a contrast medium can be injected into the main body 12a through the side arm 12b. The distal end of the main body 12a is attached to the proximal end of the guiding catheter main body 11. The main body 12a includes a proximal end opening 12c, and the balloon catheter 5 and support catheter 1 can be inserted into the main body 12a through the proximal end opening 12c.

### <Balloon catheter>

The balloon catheter 5 is a therapeutic catheter, and any known balloon catheter may be used as the balloon catheter 5. The balloon catheter 5 is inserted into the stenosed region 3 of the coronary artery to dilate the stenosed region 3. The balloon catheter 5 is, for example, a rapid exchange (RX) catheter. As shown in FIG. 1, the balloon catheter 5 includes a therapeutic catheter main body 21 and a connector 22. The therapeutic catheter main body 21 is shaped as an elongated tube. The therapeutic catheter main body 21 includes a balloon 23 at its distal end, and a stent 24 is fitted around the balloon 23. The balloon catheter 5 is used with the guide wire 25, guiding catheter 4, and support catheter 1.

### <Method for using support catheter>

Hereinafter, the way of approaching the stenosed region through the radial artery in the PCI will be described with reference to FIG. 1. The detailed structure of the support catheter 1 in the present embodiment will be described later.

The PCI is performed using the support catheter 1, guiding catheter 4, balloon catheter 5, and guide wire 25. In the PCI, the practitioner first punctures the radial artery 8 with a needle and inserts the sheath 7 into the punctured site. Subsequently, the guiding catheter 4 is inserted into the radial artery 8 through the sheath 7, and then the guiding catheter 4 is advanced until its distal end opening 4a reaches the inlet 2a of the coronary artery 2 through an aortic arch 9. Once the distal end opening 4a reaches the inlet 2a, the guide wire 25 is inserted, and the support catheter 1 is inserted through the proximal end opening 4b of the guiding catheter 4. The support catheter 1 is advanced inside the guiding catheter 4 while being pushed or pulled by the practitioner and guided by the guide wire 25 until the distal portion of the support catheter 1 projects out of the distal end opening 4a. Thus, the distal portion of the support catheter 1 is inserted into the coronary artery 2 and finally reaches the stenosed region 3.

After the distal portion of the support catheter 1 is pushed into the stenosed region 3 as described above, the balloon catheter 5 is inserted through the proximal end opening 4b of the guiding catheter 4. The balloon catheter 5 is advanced until its distal end is inserted into a distal shaft 33 of the support catheter 1 and then projects out of the distal end of the distal shaft 33. Advancing the balloon catheter 5 in this manner leads to the distal portion of the balloon catheter 5 being inserted into the stenosed region 3 and to the balloon 23 and stent 24 being placed in the stenosed region 3. The advancement of the balloon catheter 5 is then stopped.

As the balloon catheter 5 is advanced as described above, the distal portion of the balloon catheter 5 is guided to the inlet 2a of the coronary artery 2 by the guiding catheter 4 and then guided to the stenosed region 3 by the support catheter 1. Since the distal shaft 33 extends up to or close to the stenosed region 3, the distal portion of the balloon catheter 5 is supported by the distal portion of the distal shaft 33 when pushed into the stenosed region 3. After that, the balloon 23 is inflated with a pressure fluid. Along with inflation, the stent 24 is expanded to dilate the stenosed region 3. In this manner, the blood flow through the stenosed region 3 can be recovered. The above-described method for using the support catheter 1 according to the first disclosure with the guiding catheter 4 and balloon catheter 5 applies also to a support catheter 1000 according to the second disclosure described later.

### <Support catheter>

The following describes the structure of the support catheter 1 of the present embodiment. As described above, the support catheter 1 is a catheter advanced to a point near the stenosed region 3 to guide the balloon 23 of the balloon catheter 5 to the stenosed region 3. The support catheter 1 serves also to support the balloon 23 when the balloon 23 is inserted into the stenosed region 3. The support catheter 1 is long enough to project out of the distal end opening 4a of the guiding catheter 4 when inserted into the guiding catheter 4 through the proximal end opening 4b. The same applies to the support catheter 1000 according to the second disclosure described later.

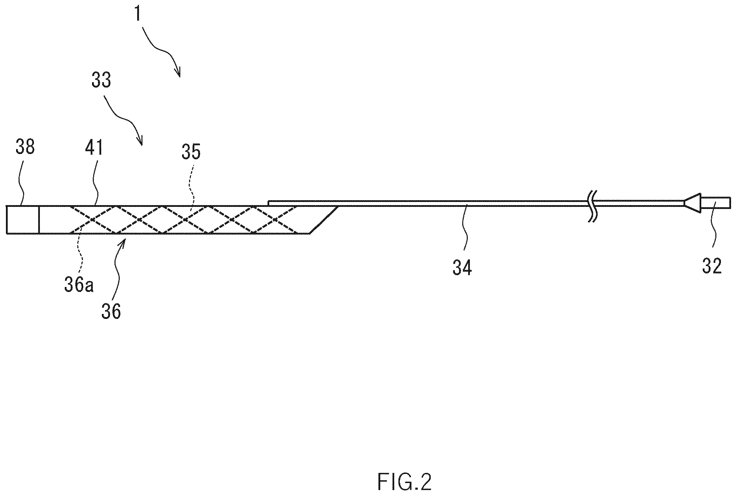

As shown in FIG. 2, the support catheter 1 includes a protective member 32, a distal shaft 33, and a proximal shaft 34 connected to the distal shaft 33.

The proximal shaft 34 is, for example, an elongated, wire-shaped member made of a metal such as stainless steel or a synthetic resin such as polyimide or polyether ether ketone. The surface of the proximal shaft 34 is coated, for example, with PTFE. The protective member 32 is located at the proximal end of the proximal shaft 34. The protective member 32 is shaped as a solid cylinder and made, for example, of a polyamide elastomer.

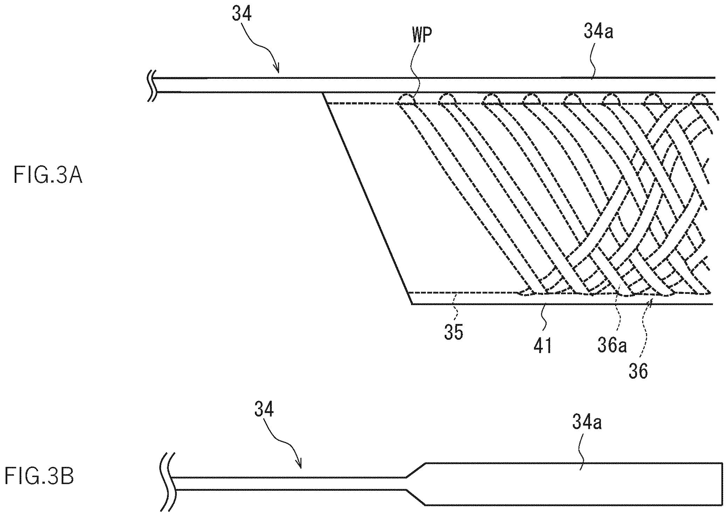

The distal shaft 33 is shaped substantially as a cylindrical tube and adapted to receive insertion of the balloon catheter 5. The distal shaft 33 includes an inner layer 35, a reinforcing layer 36, and an outer layer 41 as shown in FIG. 2 and FIGS. 3A and 3B.

The inner layer 35 of the distal shaft 33 is made, for example, of polytetrafluoroethylene (PTFE) or tetrafluoroethylene-perfluoroalkyl vinyl ether copolymer (PFA). The inner layer 35 is produced, for example, by applying PTFE to the outer surface of a silver-plated copper wire. The material of the inner layer 35 is not limited to those mentioned above.

The reinforcing layer 36 of the distal shaft 33 is shaped as a tubular mesh (tubular net) including metal wires (wires) 36a made of a metal such as stainless steel and wound in first and second opposite directions. The reinforcing layer 36 is located on the outer circumferential surface of the inner layer 35.

The outer layer 41 of the distal shaft 33 is shaped substantially as a cylindrical tube and made, for example, of a nylon-based elastomer resin or polybutylene terephthalate. Preferably, the proximal end of the outer layer 41 is cut obliquely as shown in FIG. 2 or is arc-shaped or half moon-shaped. In this case, the proximal end of the inner layer 35 preferably has the same shape as the proximal end of the outer layer 41. This facilitates insertion of the balloon catheter 5 into the distal shaft 33. The distal shaft 33 is not limited to having one outer layer and may include two or more outer layers. The material of the outer layer 41 is not limited to those mentioned above.

The inner and outer layers 35 and 41 may be made of the same material, which is not limited to the materials mentioned above. The outer circumferential surface of the outer layer 37 may be coated with a hydrophilic polymer containing polyurethane or polyvinylpyrrolidone (PVP).

A distal tip 38 is located at the distal end of the distal shaft 33. The distal tip 38 is made of a polyamide elastomer containing a material such as bismuth oxide serving as a contrast medium and is shaped substantially as a cylindrical tube. The distal tip 38 is radiopaque and casts a shadow in radiological images.

Hereinafter, the reinforcing layer 36 located on the outer circumferential surface of the inner layer 35 of the distal shaft 33 will be described in detail.

Referring to FIG. 3A, for example, 16 metal wires 36a are used to form the reinforcing layer 36. The reinforcing layer 36 can be formed by helically winding eight wires of the 16 metal wires 36a around the outer circumferential surface of the inner layer 35 in the first direction and helically winding the other eight wires 36a around the outer circumferential surface of the inner layer 35 in the second direction.

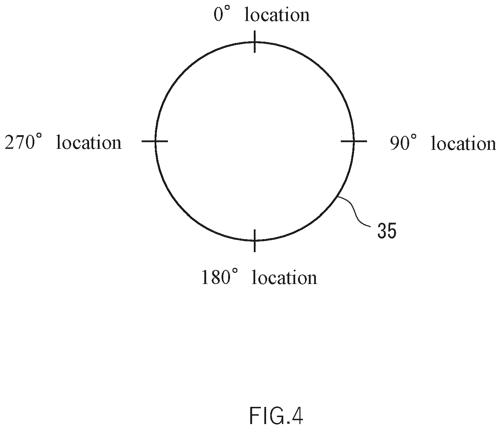

In the case where the reinforcing layer 36 is formed using 16 metal wires 36a as described above, the metal wires 36a wound in the first direction and the metal wires 36a wound in the second direction are welded at eight intersections thereof to form eight welds WP arranged in a single row extending in the axial direction of the distal shaft 33, and then the ends of the metal wires 36a are cut by laser beams. Alternatively, the ends of the metal wires 36a may be cut by laser beams at the same time as the welds WP are formed in the above manner. Thus, eight welds WP arranged in a single row extending in the axial direction of the distal shaft 33 are formed while the metal wires 36a are prevented from being radially expanded upon cutting of their ends. The welds WP thus formed are substantially ball-shaped or convex. As shown in FIG. 4, locations on the inner layer 35 that are 90° away from one another in the circumferential direction of the inner layer 35 and that extend in the longitudinal direction of the inner layer 35 are defined as 0°, 90°, 180°, and 270° locations. In FIG. 3A, the eight welds WP are formed at intersections of the metal wires 36a intersecting, for example, at the 0° location. At the 0° location, the eight welds WP are arranged in a single row extending in the axial direction of the distal shaft 33.

When, as described above, the welds WP are located at the 0° location and arranged in a single row extending in the axial direction of the distal shaft 33, the area over which the welds WP are covered by a covering member can be reduced. Specifically, the distal portion 34a of the proximal shaft 34, which is shown in FIG. 3B, can be used as the covering member. The distal portion 34a of the proximal shaft 34 is wider than the rest of the proximal shaft 34. This distal portion 34a of the proximal shaft 34 is welded over the eight welds WP located at the 0° location. Thus, the eight welds WP at the 0° location can be covered by the distal portion 34a of the proximal shaft 34.

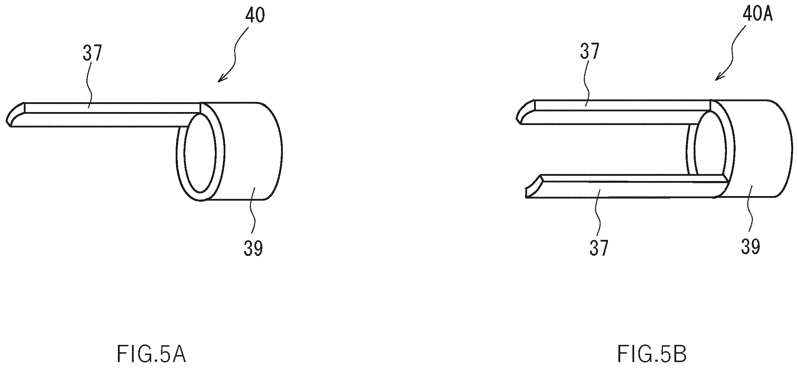

Alternatively, as described in detail below, the welds WP may be covered by using a cover piece 37 as the covering member. As shown in FIG. 5A, a covering structure 40 including a tubular member 39 and a cover piece 37 extending from one end surface of the tubular member 39 is prepared. The covering structure 40 is made, for example, of a nylon-based elastomer resin. The material of the covering structure 40 is not limited to that mentioned above. The cover piece 37 as viewed in the axial direction of the tubular member 39 is arc-shaped. A double-layered portion made up of the outer layer 41 and inner layer 35 (double-layered portion that does not include the reinforcing layer 36) is inserted into the hole of the tubular member 39 of the covering structure 40, and in this state positioning is made between the distal shaft 33 and the covering structure 40. The cover piece 37 is placed and welded over the eight welds WP located at the 0° location. The welding is followed by cutting the tubular member 39 with the double-layered portion inserted therein. When this way of covering is used, the distal shaft 33 is more flexible than when the welds WP are covered by the distal portion 34a of the proximal shaft 34. The eight welds WP at the 0° location can be covered also in the manner as described above.

In the support catheter 1 of the present embodiment, as descried above, the welds WP can be located in a limited region in the circumferential direction of the distal shaft 33; in particular, the welds WP can be located at the 0° location and arranged in a single row extending in the axial direction of the distal shaft 33. Additionally, the welds WP can be covered by the distal portion 34a of the proximal shaft 34. Thus, radial sticking out of the welds WP from the outer layer 41 can be reduced or avoided. Further, unlike in conventional structures, there is no need for any intermediate layer located between the inner and outer layers and extending over the entire circumference of the distal shaft, and the distal portion 34a of the proximal shaft 34, which serves as a covering member, is placed only over the limited region in which the welds WP are located. This can reduce the increase in diameter of the distal shaft 33, and at the same time ensure the flexibility of the distal shaft 33 to make the distal shaft 33 easily bendable.

### (Second embodiment according to first disclosure)

The second embodiment will be described with primary emphasis on differences from the first embodiment. The same applies to the third and subsequent embodiments described later.

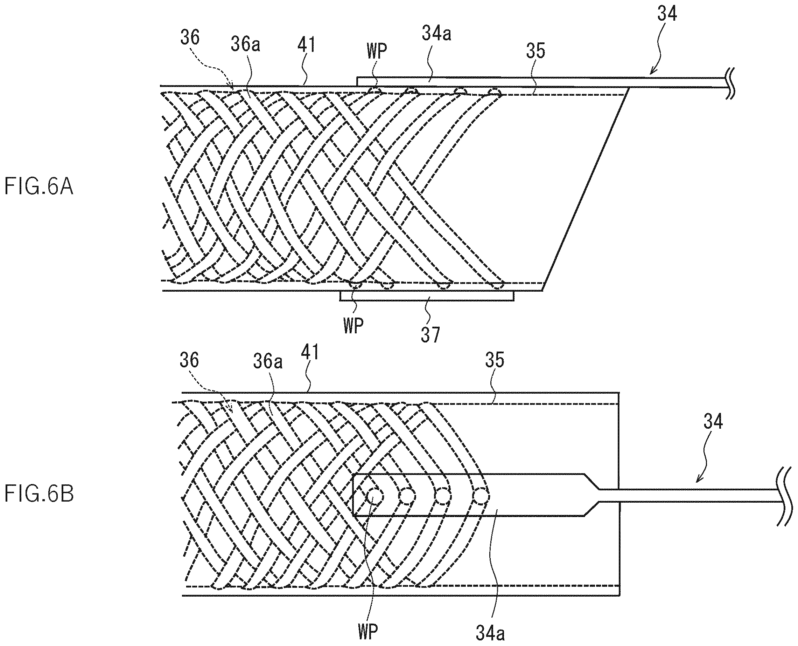

In the present embodiment, as shown in FIG. 6A, the metal wires 36a intersecting, for example, at the 0° and 180° locations are welded at their intersections, and thus the welds WP are formed. In the example of FIG. 6A, four of the welds WP are located at the 0° location and arranged in a row extending in the axial direction of the distal shaft 33, and the other four welds WP are located at the 180° location and arranged in a row extending in the axial direction of the distal shaft 33.

When, as described above, the welds WP are located at the 0° and 180° locations and are arranged in rows extending in the axial direction of the distal shaft 33, each of the areas over which the welds WP are covered can be reduced. As in the method described above with reference to FIG. 5A and as shown in FIG. 6A, the cover piece 37 of the covering structure 40 is placed and welded over the four welds WP located at one of the 0° and 180° locations. Further, as shown in FIGS. 6A and 6B, the distal portion 34a of the proximal shaft 34 is welded over the four welds WP located at the other of the 0° and 180° locations. In this manner, the welds WP at the 0° and 180° locations can be covered by the cover piece 37 and the distal portion 34a of the proximal shaft 34.

Alternatively, the welds WP may be covered in the following way. As shown in FIG. 5B, a covering structure 40A is prepared which includes a tubular member 39 and a pair of cover pieces 37 extending from diametrically opposite circumferential segments of one end surface of the tubular member 39. The covering structure 40A is made of the same material as the covering structure 40 described above. The cover pieces 37 as viewed in the axial direction of the tubular member 39 are arc-shaped. As in the method described above for the covering structure 40, one of the cover pieces 37 of the covering structure 40A is placed and welded over the four welds WP located at the 0° location and the other cover piece 37 is placed and welded over the four welds WP located at the 180° location. In this case, the distal shaft 33 is more flexible than in the case where the welds WP are covered by the distal portion 34a of the proximal shaft 34.

In the support catheter 1 of the present embodiment, as descried above, the welds WP can be located in limited regions in the circumferential direction of the distal shaft 33; in particular, the welds WP can be located at the 0° and 180° locations and thus arranged in two rows extending in the axial direction of the distal shaft 33. Additionally, the welds WP at each of the 0° and 180° locations can be covered by the cover piece 37 or the distal portion 34a of the proximal shaft 34. This makes the welds WP less likely to stick out from the outer layer. Unlike in conventional structures which include an intermediate layer located between the inner and outer layers and extending over the entire circumference of the distal shaft, the increase in diameter of the distal shaft 33 can be reduced. Further, since the welds WP are arranged in two separate rows, the lengths of the covering members in the axial direction of the distal shaft 33, namely the lengths of the cover piece 37 and the distal portion 34a of the proximal shaft 34 in the axial direction can be reduced. This can ensure the flexibility of the distal shaft 33. Additionally, the hardness of the distal shaft 33 can be adjusted by adjusting the areas over which the distal portion 34a of the proximal shaft 34 and the cover piece 37 are located, such as by placing the distal portion 34a of the proximal shaft 34 outside some of the welds WP and placing the cover piece 37 outside the other welds WP.

### (Third embodiment according to first disclosure)

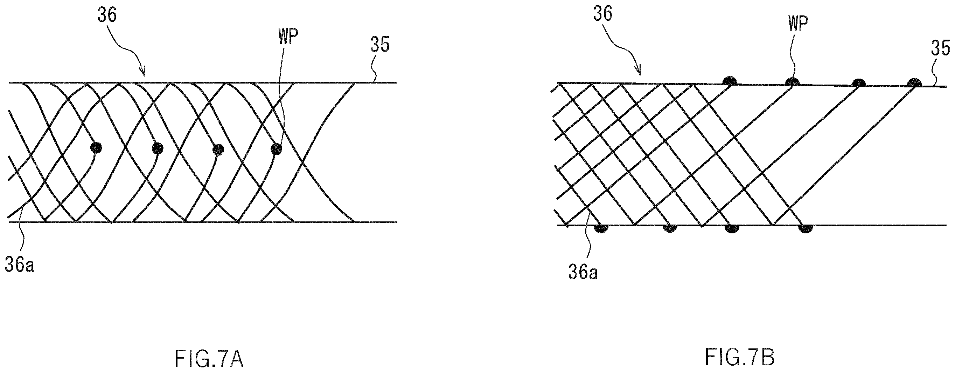

For the third embodiment, the metal wires 36a are briefly depicted in the drawings, and the outer layer 41, proximal shaft 34, and cover piece 37 are omitted in the drawings. The same applies to the fourth and subsequent embodiments described later. In the present embodiment, as shown in FIG. 7A, the metal wires 36a intersecting, for example, at the 0° and 180° locations are welded at every two intersections of a plurality of intersections aligned in the axial direction of the distal shaft 33, and thus the welds WP are formed. As in the second embodiment, the cover piece 37 of the covering structure 40 is placed and welded over the four welds WP located at one of the 0° and 180° locations, and the distal portion 34a of the proximal shaft 34 is welded over the four welds WP located at the other of the 0° and 180° locations. The welds WP can be covered in this manner. Alternatively, the welds WP may be covered by placing and welding one of the pair of cover pieces 37 of the covering structure 40A over the four welds WP located at the 0° location and placing and welding the other cover piece 37 over the four welds WP located at the 180° location.

In the present embodiment, as in the second embodiment, the welds WP are less likely to stick out from the outer layer. Additionally, unlike in conventional structures which include an intermediate layer located between the inner and outer layers and extending over the entire circumference of the distal shaft, the increase in diameter of the distal shaft 33 can be reduced. Further, since the number of the metal wires 36a forming the reinforcing layer 36 decreases gradually, an abrupt hardness change of the distal shaft 33 can be avoided.

### (Fourth embodiment according to first disclosure)

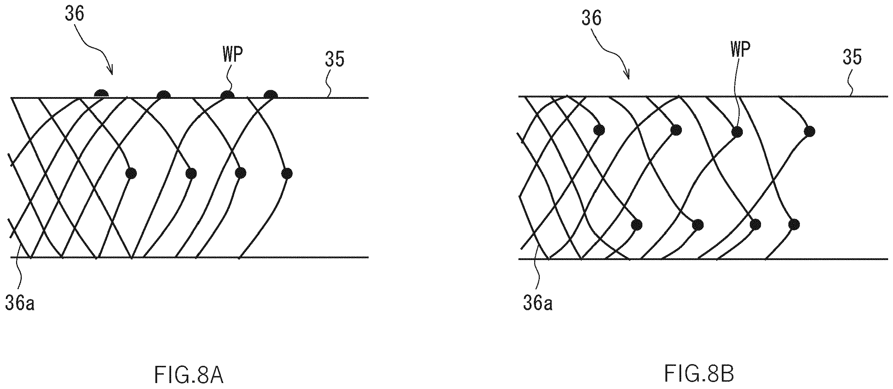

In the present embodiment, as shown in FIG. 8A, the metal wires 36a intersecting, for example, at the 0° and 90° locations are welded at their intersections, and thus the welds WP are formed. As in the second embodiment, the cover piece 37 of the covering structure 40 is placed and welded over the eight welds WP located at the 0° and 90° locations. Thus, all of the eight welds WP can be covered by the cover piece 37. Alternatively, all of the eight welds WP may be covered by the distal portion 34a of the proximal shaft 34. Alternatively, the welds WP may be covered by placing and welding the cover piece 37 of the covering structure 40 over the four welds WP located at one of the 0° and 90° locations and placing and welding the distal portion 34a of the proximal shaft 34 over the four welds WP located at the other of the 0° and 90° locations.

In the present embodiment, as in the second embodiment, the welds WP are less likely to stick out from the outer layer. Additionally, unlike in conventional structures which include an intermediate layer located between the inner and outer layers and extending over the entire circumference of the distal shaft, the increase in diameter of the distal shaft 33 can be reduced.

### (Fifth embodiment according to first disclosure)

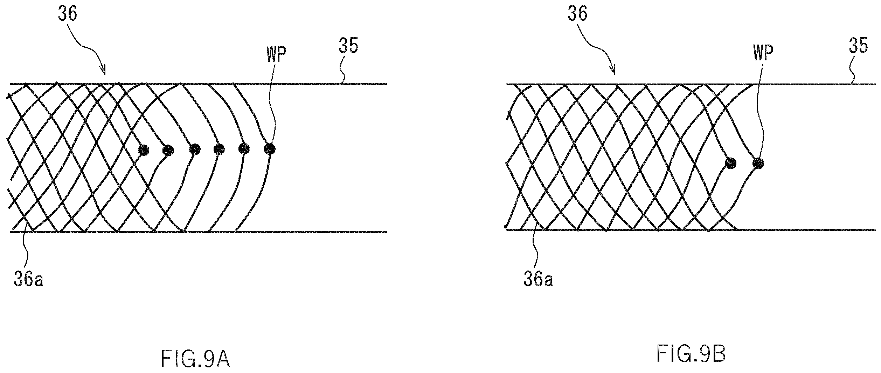

The welds WP in the present embodiment include: six welds shown in FIG. 9A that are formed as a result of welding at six intersections of the metal wires 36a intersecting, for example, at the 0° location; and two welds shown in FIG. 9B that are formed as a result of welding at two intersections of the metal wires 36a intersecting at the 180° location. As in the second embodiment, the cover piece 37 of the covering structure 40 is placed and welded over the welds WP located at one of the 0° and 180° locations. Further, the distal portion 34a of the proximal shaft 34 is welded over the welds WP located at the other of the 0° and 180° locations. In this manner, the welds WP at the 0° and 180° locations can be covered by the cover piece 37 and the distal portion 34a of the proximal shaft 34. Alternatively, the welds WP may be covered by placing and welding one of the pair of cover pieces 37 of the covering structure 40A over the six welds WP located at the 0° location and placing and welding the other cover piece 37 over the two welds WP located at the 180° location.

In the present embodiment, as in the second embodiment, the welds WP are less likely to stick out from the outer layer. Additionally, unlike in conventional structures which include an intermediate layer located between the inner and outer layers and extending over the entire circumference of the distal shaft, the increase in diameter of the distal shaft 33 can be reduced.

### (Sixth embodiment according to first disclosure)

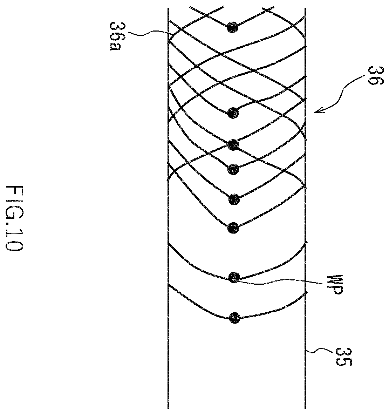

In the present embodiment, as shown in FIG. 10, the metal wires 36a intersecting, for example, only at the 0° location are welded at their intersections such that some of the welds WP are located at intersections directly next to one another in the axial direction of the distal shaft 33 and the other welds WP are located at every two or more intersections in the axial direction of the distal shaft 33. The distal portion 34a of the proximal shaft 34 is welded over these welds WP, and thus all of the welds WP can be covered. In this case, the need for any cover piece is eliminated. Alternatively, the cover piece 37 of the covering structure 40 may be placed and welded over the welds WP to cover all of the welds WP.

In the present embodiment, as in the first embodiment, the welds WP are less likely to stick out from the outer layer. Additionally, unlike in conventional structures which include an intermediate layer located between the inner and outer layers and extending over the entire circumference of the distal shaft, the increase in diameter of the distal shaft 33 can be reduced. Further, since the number of the metal wires 36a forming the reinforcing layer 36 decreases gradually, an abrupt hardness change of the distal shaft 33 can be avoided. In addition, the distal portion 34a of the proximal shaft 34 or the cover piece 37, which serves as a covering member, is placed only over the limited region in which the welds WP are located. This can ensure the flexibility of the distal shaft 33 to make the distal shaft 33 easily bendable.

### (Other embodiments according to first disclosure)

The present invention is not limited to the embodiments described above, and various modifications can be made without departing from the gist of the present invention. Examples of the modifications will be described below.

Although the reinforcing layer 36 is formed using 16 metal wires 36a in the above embodiments, the present invention is not limited to this number of the metal wires 36a. The reinforcing layer 36 may be formed using less than 16 metal wires 36a or more than 16 metal wires 36a. Additionally, the manner in which the metal wires 36a forming the reinforcing layer 36 are braided is not limited to that as shown in FIG. 3. For example, a bundle of metal wires and another bundle of metal wires may be woven together although one metal wire wound in the first direction and another metal wire wound in the second direction are woven together in the braid of FIG. 3.

Although the distal shaft 33 includes the outer layer 41 in the above embodiments, the outer layer 41 is not an essential element. In the case where the distal shaft 33 does not include the outer layer 41, the welds WP are covered directly by the distal portion 34a of the proximal shaft 34 or the cover piece 37.

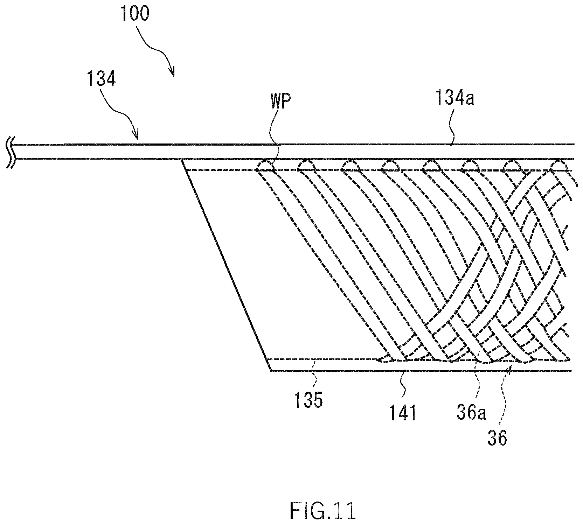

Although in the support catheter 1 of each of the above embodiments, the welds WP are located in a limited region in the circumferential direction of the distal shaft 33, the present invention is not limited to this arrangement of welds, and welds may be located in a limited region in the circumferential direction of a reinforcing layer of a tube. Specifically, as shown in FIG. 11, a tube 100 includes: a tubular inner layer 135; a reinforcing layer 36 located on the outer surface of the inner layer 135, the reinforcing layer 36 being shaped as a tubular mesh including metal wires 36a wound in first and second opposite directions; and a tubular outer layer 141. As previously described, the reinforcing layer 36 includes welds WP at intersections of the metal wires 36a wound in the first and second directions. A covering member 134 is located outside the welds WP. The covering portion 134a of the covering member 134 may be wide. The welds WP are located in a limited region in the circumferential direction of the reinforcing layer 36. The welds WP are arranged in a single row extending in the axial direction of the inner layer 135. The outer layer 141 is not an essential element. Such a tube 100 has the same advantages as the support catheter 1 described above.

### Hereinafter, embodiments of the second disclosure will be described.

### <Support catheter>

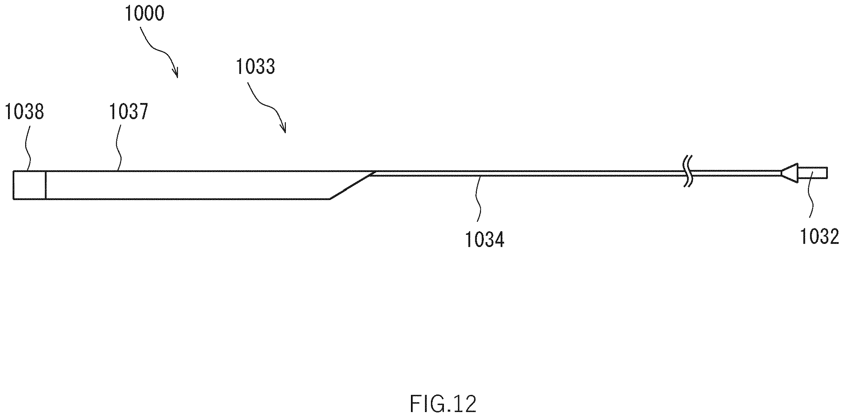

Referring to FIG. 12, a support catheter 1000 includes a protective member 1032, a distal shaft 1033, and a proximal shaft 1034 connected to the distal shaft 1033.

The proximal shaft 1034 is, for example, an elongated, wire-shaped member made of a metal such as stainless steel or a synthetic resin such as polyimide or polyether ether ketone. The surface of the proximal shaft 1034 is coated, for example, with PTFE. The protective member 1032 is located at the proximal end of the proximal shaft 1034. The protective member 1032 is shaped as a solid cylinder and made, for example, of a polyamide elastomer.

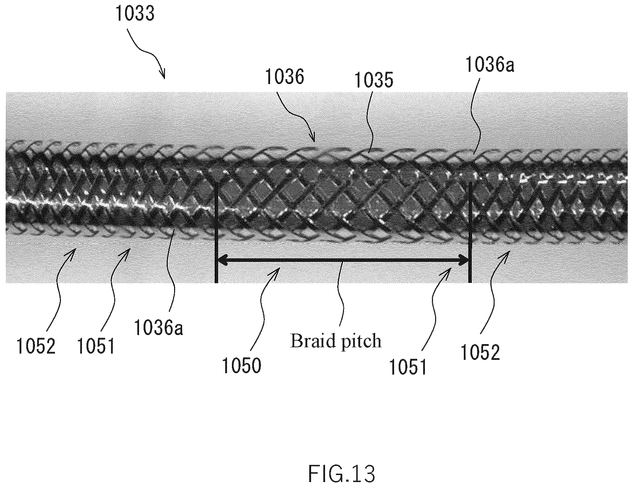

The distal shaft 1033 is shaped substantially as a cylindrical tube and adapted to receive insertion of the balloon catheter 5. As shown in FIG. 13, the distal shaft 1033 is a three-layered member including an inner layer 1035, a reinforcing layer 1036, and an outer layer 1037 (FIG. 12) arranged in order from inside to outside.

The inner layer 1035 of the distal shaft 1033 is made, for example, of polytetrafluoroethylene (PTFE) or tetrafluoroethylene-perfluoroalkyl vinyl ether copolymer (PFA). The inner layer 1035 is produced, for example, by applying PTFE to the outer surface of a silver-plated copper wire. The material of the inner layer 1035 is not limited to those mentioned above.

The reinforcing layer 1036 of the distal shaft 1033 is shaped as a tubular mesh (tubular net) including metal wires (wires) 1036a made of a metal such as stainless steel and wound in first and second opposite directions. The reinforcing layer 1036 is located on the outer circumferential surface of the inner layer 1035.

The outer layer 1037 of the distal shaft 1033 is shaped substantially as a cylindrical tube and made, for example, of a nylon-based elastomer resin or polybutylene terephthalate. Preferably, the proximal end of the outer layer 1037 is cut obliquely as shown in FIG. 12 or is arc-shaped or half moon-shaped. In this case, the proximal end of the inner layer 1035 preferably has the same shape as the proximal end of the outer layer 1037. This facilitates insertion of the balloon catheter 5 into the distal shaft 1033. The distal shaft 1033 is not limited to having one outer layer and may include two or more outer layers. The material of the outer layer 1037 is not limited to those mentioned above.

The inner and outer layers 1035 and 1037 may be made of the same material, which is not limited to the materials mentioned above. The outer circumferential surface of the outer layer 1037 may be coated with a hydrophilic polymer containing polyurethane or polyvinylpyrrolidone (PVP).

A distal tip 1038 is located at the distal end of the distal shaft 33. The distal tip 1038 is made of a polyamide elastomer containing a material such as bismuth oxide serving as a contrast medium and is shaped substantially as a cylindrical tube. The distal tip 1038 is radiopaque and casts a shadow in radiological images.

Hereinafter, the reinforcing layer 1036 located on the outer circumferential surface of the inner layer 1035 of the distal shaft 1033 will be described in detail.

For example, 16 metal wires 1036a are used to form the reinforcing layer 1036. The reinforcing layer 1036 can be formed by helically winding eight wires of the 16 metal wires 1036a around the outer circumferential surface of the inner layer 35 in the first direction and helically winding the other eight wires 1036a around the outer circumferential surface of the inner layer 35 in the second direction. The number of the metal wires 1036a wound in the first direction and the number of the metal wires 1036a wound in the second direction are not limited to eight. The metal wires 1036a are not limited to being wound helically and may be wound in any known manner.

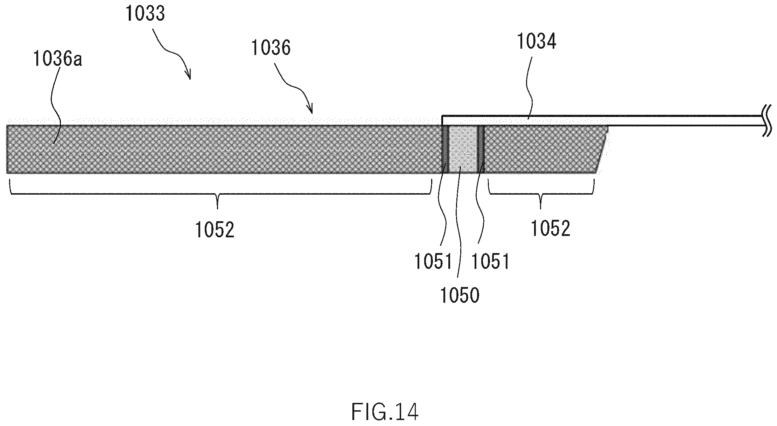

As shown in FIGS. 13 and 14, the reinforcing layer 1036 includes a long pitch portion 1050, two pitch-changing portions 1051, and two short pitch portions 1052. For example, the long pitch portion 1050 may have a length of 1 to 30 mm, the distal short pitch portion 1052 may have a length of 200 to 500 mm, the proximal short pitch portion 1052 may have a length of 0 to 30 mm, and each pitch-changing portion 1051 may have a length of 1 to 5 mm. The lengths of these portions are not limited to the mentioned ranges and may be set appropriately depending on, for example, the blood vessels in which the support catheter 1000 is used. The pitch-changing portion 1051 is preferably short in order to ensure the flexibility of the distal shaft and preferably long in order to moderate the change in hardness of the distal shaft.

The long pitch portion 1050 is located between the two short pitch portions 1052. One of the pitch-changing portions 1051 is located between one of the short pitch portions 1052 and the long pitch portion 1050, and the other pitch-changing portion 1051 is located between the other short pitch portion 1052 and the long pitch portion 1050.

The short pitch portion 1052 is a portion in which the braid pitch (pitch) is a first value. The braid pitch is the distance between two portions of each metal wire 1036a wound in the first direction (or the second direction), the two portions being at the same angular location in the circumferential direction of the reinforcing layer 1036 (the location may be any angular location and may be, for example, the 0° location) and adjacent to each other in the axial direction. That is, the braid pitch may be the distance from one portion of the wound metal wire 1036a that is located at the 0° location to another portion of the wound metal wire 1036a that is also located at the 0° location and that is next to the one portion. The first value is not limited to a particular range and may be any value smaller than a second value of the braid pitch in the long pitch portion 1050 described below. The first value can be set appropriately depending on, for example, the blood vessels in which the support catheter 1000 is used.

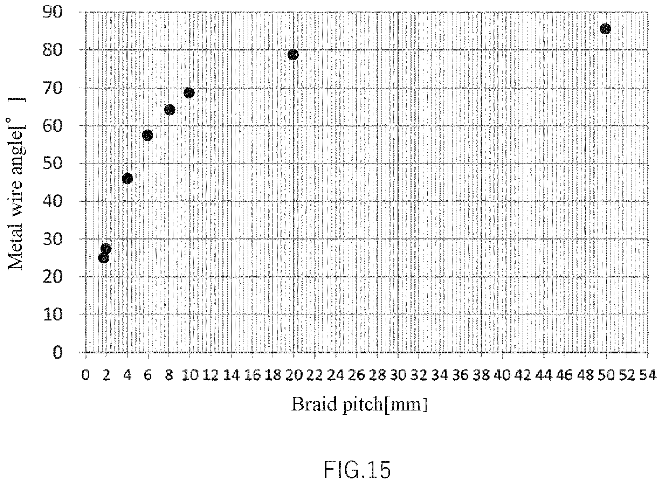

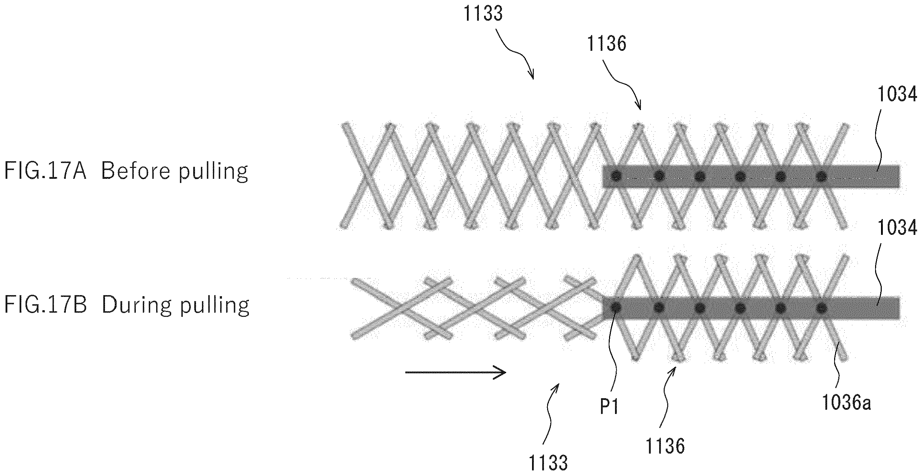

The long pitch portion 1050 is a portion in which the braid pitch is a second value greater than the first value. As shown in FIG. 17A described later, when the support catheter 1000 is in a normal state, an acute angle (metal wire angle) α between one metal wire 1036a of the long pitch portion 1050 and a straight line L perpendicular to the longitudinal direction of the proximal shaft 1034 is, for example, from 25° to 70° in plan view. When the angle α is in this range, the second value can, for example, be in the range of 2 to 10 mm as shown in FIG. 15. The normal state of the support catheter 1000 is defined herein as a state where the support catheter 100 is contained in a package or a state where the support catheter 100 has been taken out of the package but is unused and free from any load.

The pitch-changing portion 1051 is a portion in which the braid pitch decreases in a direction from the long pitch portion 1050 to the short pitch portion 1052. For example, when the braid pitch in the long pitch portion 1050 is 6 mm and the braid pitch in the short pitch portion 1052 is 2 mm, the braid pitch in the pitch-changing portion 1051 gradually changes from 6 mm to 2 mm.

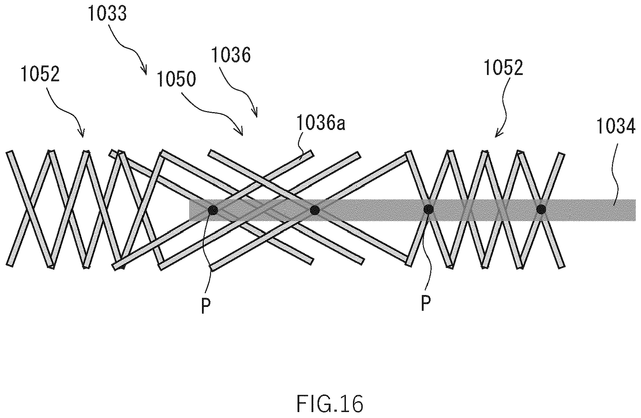

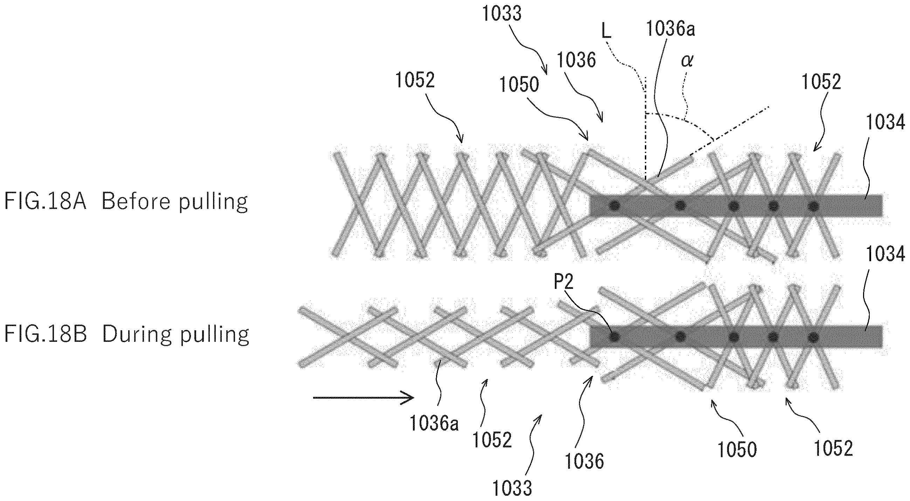

In the present embodiment, one end portion of the proximal shaft 1034 is welded to the long pitch portion 1050 and the proximal short pitch portion 1052. Specifically, one end portion of the proximal shaft 1034 is fixed to the long pitch portion 1050 at two or more of axially aligned intersections of the metal wires 1036a wound in the first and second directions. The fixing method may be, but is not limited to, welding. In this case, as shown in FIG. 16, the one end portion of the proximal shaft 1034 is welded to the long pitch portion 1050 at every two intersections of the axially aligned intersections. The letters P in FIG. 16 represent the welds. The one end portion of the proximal shaft 1034 is welded to the short pitch portion 1052 at every three or more intersections of the axially aligned intersections. As shown in FIG. 18A described later, the one end portion of the proximal shaft 1034 may be welded to the long pitch portion 1050 and the short pitch portion 1052 at every one of the axially aligned intersections.

Hereinafter, the features and advantages of the support catheter 1000 of the present embodiment will be described with reference to FIGS. 17 and 18. FIGS. 17 and 18 are schematic diagrams simplified in favor of ease of understanding of the features and advantages over exact depiction. FIG. 17A shows a conventional distal shaft 1133 including a reinforcing layer (conventional reinforcing layer) 1136 in the form of a braid having a constant braid pitch over its entirety. When the conventional distal shaft 1133 is pulled in the direction of the arrow of FIG. 17B, the portion of the braid 1136 that is not fixed by the proximal shaft 1034 is gradually deformed in such a direction that the braid pitch increases (the angle of the metal wires 1036a increases), but the portion of the braid 1136 that maintains a fixed angle of the metal wires 1036a cannot conform to the above deformation. Thus, a load is imposed on a fixing point P1 which is at the boundary between the pitch variable portion where the braid pitch changes and the pitch invariable portion where the pitch remains unchanged. This is likely to lead to detachment of the one end portion of the proximal shaft 1034 from the metal wires 1036a.

In the support catheter 1000 of the present embodiment, the long pitch portion 1050 of the distal shaft 1033 has a longer braid pitch than the rest of the distal shaft 1033. Thus, even when the distal shaft 1033 of the present embodiment, which is shown in FIG. 18A, is pulled in the direction of the arrow of FIG. 18B, the load imposed on a fixing point P2 due to changes occurring in the portion of the braid 1036 that is not fixed by the proximal shaft 1034 (changes in the braid pitch and in the angle of the metal wires 1036a) is reduced. This makes the one end portion of the proximal shaft 1034 resistant to detachment from the metal wires 1036a. The pitch-changing portions 1051 are omitted in FIG. 18.

In the support catheter 1000 of the present embodiment, as described above, the reinforcing layer 1036 includes the long pitch portion 1050, and one end portion of the proximal shaft 1034 is fixed to the long pitch portion 1050. Thus, in the event that a longitudinal tensile force acts on the distal shaft 1034, the load imposed on the fixing points at which the one end portion of the proximal shaft 1034 is fixed to the long pitch portion 1050 can be lower than in the case where the one end portion of the proximal shaft 1034 is fixed only to the short pitch portion 1052. This makes the one end portion of the proximal shaft 1034 resistant to detachment from the metal wires 1036a.

In the present embodiment, each pitch-changing portion 1051 is located between the long pitch portion 1050 and a corresponding one of the short pitch portions 1052, and the braid pitch changes gradually between the long pitch portion 1050 and each short pitch portion 1052. Thus, an abrupt hardness change of the distal shaft 1033 can be avoided to prevent breakage of the distal shaft 1033.

In the present embodiment, the proximal shaft 1034 is welded to the long pitch portion 1050 and the proximal short pitch portion 1052, and thus the area of welding of the proximal shaft 1034 can be increased. This can reduce the likelihood of detachment of the proximal shaft 1034.

In the present embodiment, the one end portion of the proximal shaft 1034 is fixed to the long pitch portion 1050 at two or more points. Thus, even in the event that the one end portion of the proximal shaft 1034 is detached from the distal fixing point subjected to the highest load, the proximal shaft 1034 and the distal shaft 1033 are not readily separated from each other.

In the present embodiment, the one end portion of the proximal shaft 1034 is welded to the long pitch portion 1050 at every two intersections of the axially aligned intersections and welded to the short pitch portion 1052 at every three or more intersections of the axially aligned intersections. In this case, the catheter manufacturing is easier than in the case where the one end portion of the proximal shaft 1034 is welded to the long pitch portion 1050 and the short pitch portion 1052 at every one of the axially aligned intersections.

In the present embodiment, the acute angle α in the long pitch portion 1050 is from 25° to 70°. The closer the acute angle α is to 90°, the higher the longitudinal tensile strength of the distal shaft 1033 is. The closer the acute angle α is to 0°, the higher the flexibility of the distal shaft 1033 is, and the easier it is to move the distal shaft 1033 radially.

### (Other embodiments according to second disclosure)

The present invention is not limited to the embodiment described above, and various modifications can be made without departing from the gist of the present invention. Examples of the modifications will be described below.

Although the pitch-changing portions 1051 are located between one of the short pitch portions 1052 and the long pitch portion 1050 and between the other short pitch portion 1052 and the long pitch portion 1050 in the above embodiment, the present invention is not limited to this arrangement of the pitch-changing portions 1051. The pitch-changing portion 1051 may be located at least between one of the short pitch portions 1052 and the long pitch portion 1050 or between the other short pitch portion 1052 and the long pitch portion 1050.

Although one end portion of the proximal shaft 1034 is fixed to the long pitch portion 1050 and proximal short pitch portion 1052 of the reinforcing layer 1036 in the above embodiment, the present invention is not limited to this manner of fixing of the one end portion of the proximal shaft 1034. The one end portion of the proximal shaft 1034 may be fixed also to the distal pitch-changing portion 1051 or may be fixed only to the long pitch portion 1050.

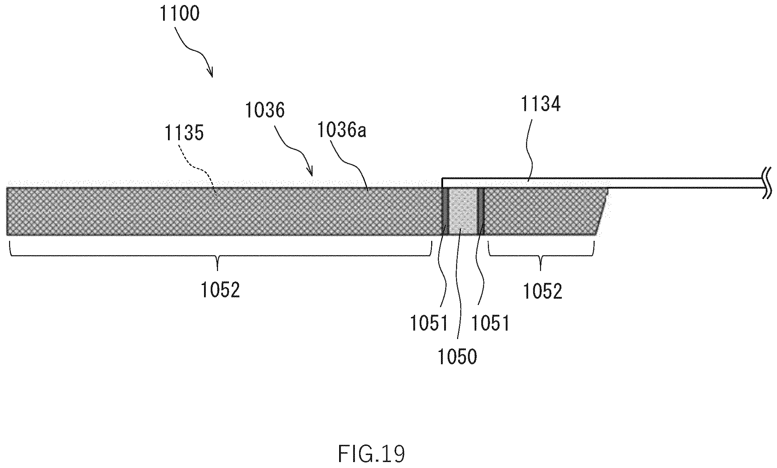

Although in the support catheter 100 of the above embodiment, one end portion of the proximal shaft 1034 is fixed to the long pitch portion 1050 of the reinforcing layer 1036, the present invention is not limited to the fixing of the one end portion of the proximal shaft 1034 to the long pitch portion 1050, and a fixed member of a tube may be fixed to a long pitch portion of a reinforcing layer of the tube. Specifically, as shown in FIG. 19, a tube 1100 includes: a tubular inner layer 1135; and a reinforcing layer 1036 shaped as a tubular mesh including metal wires 1036a wound in first and second opposite directions. As previously described, the reinforcing layer 1036 includes short pitch portions 1052, pitch-changing portions 1051, and a long pitch portion 1050. The tube 1100 further includes a fixed member 1134 fixed to the long pitch portion 1050. The tube 1100 may include a tubular outer layer located outside the reinforcing layer 1036. Such a tube 1100 has the same advantages as the support catheter 1000 described above.

In the above embodiment, the distal tip 1038 is located at the distal end of the distal shaft 1033. The distal tip 1038 may be welded to the reinforcing layer 1036 of the distal shaft 1033 and, in this case, the portion of the reinforcing layer 1036 that includes the welds may be a long pitch portion having a longer braid pitch than the rest of the reinforcing layer 1036.

### Reference Signs List

- 1: support catheter
- 4: guiding catheter
- 5: balloon catheter
- 33: distal shaft
- 34: proximal shaft
- 34a: distal portion of proximal shaft (covering member)
- 35, 135: inner layer
- 36: reinforcing layer
- 36a: metal wire
- 37: cover piece (covering member)
- 100: tube
- 134: covering member
- WP: weld

## Claims

1. A support catheter for use with a therapeutic catheter for treating a treatment site and a guiding catheter for receiving insertion of the therapeutic catheter and guiding the therapeutic catheter in a blood vessel, the support catheter being long enough to project out of a distal end opening of the guiding catheter when inserted into the guiding catheter through a proximal end opening of the guiding catheter, the support catheter being adapted to guide a distal portion of the therapeutic catheter to the treatment site, the support catheter comprising:
a distal shaft shaped as a tube into which the therapeutic catheter is insertable, the distal shaft including an inner layer and a reinforcing layer, the reinforcing layer being shaped as a tubular mesh including metal wires wound in first and second opposite directions; and
a proximal shaft connected to the distal shaft, wherein
the reinforcing layer includes welds at intersections of the metal wires wound in the first and second directions,
the welds are located in a limited region in a circumferential direction of the distal shaft, and
a covering member is located outside the welds.

2. The support catheter according to claim 1, wherein the covering member includes at least one of a cover piece and a distal portion of the proximal shaft made of a metal.

3. The support catheter according to claim 1 or 2, wherein the welds are arranged in a single row extending in an axial direction of the distal shaft.

4. The support catheter according to claim 1 or 2, wherein the welds are arranged in two rows extending in an axial direction of the distal shaft.

5. The support catheter according to claim 4, wherein one of the two rows is 180° away from the other row in a circumferential direction of the inner layer.

6. The support catheter according to claim 4, wherein one of the two rows is 90° away from the other row in a circumferential direction of the inner layer.

7. A tube comprising:
a tubular inner layer; and
a reinforcing layer located on an outer surface of the inner layer and shaped as a tubular mesh including metal wires wound in first and second opposite directions, wherein
the reinforcing layer includes welds at intersections of the metal wires wound in the first and second directions,
the welds are located in a limited region in a circumferential direction of the reinforcing layer, and
a covering member is located outside the welds.

8. The tube according to claim 7, wherein the welds are arranged in a single row extending in an axial direction of the inner layer.

9. A support catheter for use with a therapeutic catheter for treating a treatment site and a guiding catheter for receiving insertion of the therapeutic catheter and guiding the therapeutic catheter in a blood vessel, the support catheter being long enough to project out of a distal end opening of the guiding catheter when inserted into the guiding catheter through a proximal end opening of the guiding catheter, the support catheter being adapted to guide a distal portion of the therapeutic catheter to the treatment site, the support catheter comprising:
a distal shaft shaped as a tube into which the therapeutic catheter is insertable, the distal shaft including an inner layer and a reinforcing layer, the reinforcing layer being shaped as a tubular mesh including metal wires wound in first and second opposite directions; and
a proximal shaft connected to the distal shaft, wherein
the reinforcing layer includes a short pitch portion in which a pitch of the wound metal wires is a first value and a long pitch portion in which the pitch of the wound metal wires is a second value greater than the first value, and
one end portion of the proximal shaft is fixed to the long pitch portion.

10. The support catheter according to claim 9, wherein
the reinforcing layer includes two short pitch portions in which the pitch of the wound metal wires is the first value, and
the long pitch portion is located between the two short pitch portions.

11. The support catheter according to claim 9 or 10, wherein
the reinforcing layer includes a pitch-changing portion between the short pitch portion and the long pitch portion, and
in the pitch-changing portion, the pitch of the wound metal wires decreases in a direction from the long pitch portion to the short pitch portion.

12. The support catheter according to any one of claims 9 to 11, wherein the one end portion of the proximal shaft is welded to the long pitch portion at two or more of axially aligned intersections of the metal wires wound in the first and second directions.

13. The support catheter according to any one of claims 9 to 12, wherein when the support catheter is in a normal state, an acute angle between the wound metal wire of the long pitch portion and a straight line perpendicular to a longitudinal direction of the inner layer is from 25° to 70°.

14. A tube comprising:
a tubular inner layer; and
a reinforcing layer located on an outer surface of the inner layer and shaped as a tubular mesh including metal wires wound in first and second opposite directions, wherein
the reinforcing layer includes a short pitch portion in which a pitch of the wound metal wires is a first value and a long pitch portion in which the pitch of the wound metal wires is a second value greater than the first value, and
the tube further comprises a fixed member fixed to the long pitch portion.
